# EUROPEAN PATENT APPLICATION

(11) **EP 0 779 061 A2**
(43) Date of publication of application: **18.06.1997**
(21) Application number: 96309058.4
(22) Date of filing: 12.12.1996
(51) Int. Cl.: A61B 17/39

(54) **Electrosurgical instrument and method for the production therof**

(30) Priority: 15.12.1995 GB 9525652
(71) Applicant: bvba VANDERSTRAETEN E, 9800 Deinze (BE)
(72) Inventor: Vanderstraeten, Johan Emile Maria, 9031 Drongen, (BE)
(74) Representative: Allard, Susan Joyce

(57) **Abstract**

An electrosurgical instrument for use with a source of radio-frequency energy during surgery to cut or to cauterize tissue, which instrument comprises a metallic tool portion which is coated on the faces thereof, other than the edge or area which is in use during surgery, with an electrically insulating and thermally non-conductive ceramic material which is coated and/or impregnated with a viscoelastic polymer.

## Description

The present invention relates to an electrosurgical instrument and a method for the production thereof.

Electrosurgical knives or blades which are currently used by surgeons comprise a surgical steel cutting blade, for example a scalpel, and a source of radio-frequency electrical energy for application to the blade. During use, the surgeon controls the application of the radio-frequency electrical energy to the blade in order to cause haemostasis of the living tissue cut by the blade. In this manner, the surgeon can minimise bleeding during operations.

Although electrosurgical knives have proved effective for controlling bleeding during surgery, one major problem is that the tissue which is cut by the blade sticks to the knife and as the knife is continually heated the tissue becomes charred, thereby reducing the efficiency of the blade, in particular its cutting ability. It has been proposed in the prior art to coat the blade, or at least the cutting edge of the blade, with a non-stick material. Non-stick coatings for electrosurgical knives are thus disclosed in US-A-4314559, US-A-4333467, US-A-4161950 and US-A-4481057. An improved coated electrosurgical knife is disclosed in US-A-4785807 in which a plurality of coatings of a non-stick material are applied to a stainless steel blade. The first coat of non-stick material is etched prior to the application of the second coat in order to provide better adhesion between the coatings.

A disadvantage of US-A-4785807 is that the non-stick material possesses some porosity which causes electrical arcing over the overall surface.

US-A-5197962 discloses an electrosurgical instrument which has a region for contact with flesh or tissue which is at least partially coated with a composite coating consisting essentially of a nickel-phosphorus matrix having particles of polytetrafluoroethylene distributed therein. Generally the whole of the blade surfaces which come into contact with flesh or tissue are coated with the said coating by means of an electroless plating process.

GB-A-2085765 discloses an electrosurgical knife in which a copper composition, preferably an alumina dispersion strengthened copper, having a yield strength of at least 25,000 psi is laminated to a steel substrate having a cutting edge, with a non-stick coating being applied to the cutting edge and at least a portion of the blade body.

EP-A-0280798 discloses a electrosurgical instrument having a conductive blade in which a layer of insulation covers the blade, except for the cutting edge thereof. The insulation material may be a ceramic, glass or other non-conductive material.

W094/05226 discloses insulation coatings for electro-surgical devices which preferably comprise alumina.

Although partial insulation coatings for electrosurgical devices are suggested in the process art, these coatings as described are porous and when exposed to body fluids become conductive, thereby reducing their efficiency.

We have now developed an improved electrosurgical instrument comprising a metallic tool portion, in which the faces of the tool, other than the cutting edge are coated with an insulating coating impregnated with a viscoelastic polymer. The instrument possesses improved insulation of the non-working areas of the tool which results in less heating of the tool surfaces and less sticking to and charring of tissue, when in use.

Accordingly, in one aspect the present invention provides an electrosurgical instrument for use with a source of radio-frequency energy during surgery to cut or to cauterize tissue, which instrument comprises a metallic tool portion which is coated on the faces thereof, other than the edge or area which is in use during surgery, with an electrically insulating and thermally non-conductive ceramic material which is coated and/or impregnated with a viscoelastic polymer.

In another aspect the present invention provides a method for the preparation of an electrosurgical instrument for use with a source of radiofrequency energy during surgery to cut or to cauterize tissue, which method comprises coating a metallic tool portion of the instrument on the faces thereof, other than the edge or area which is in use during surgery, with an electrically insulating and thermally non-conductive ceramic material, and coating or impregnating the said coating of the ceramic material with a viscoelastic polymer.

The electrosurgical instrument of the present invention is preferably an electrosurgical knife which may comprise a surgical steel cutting blade and may, for example, be a scalpel. In the following description the invention is described with reference to an electrosurgical knife, although it will be understood that the invention is generally applicable to other electrosurgical instruments.

In producing the electrosurgical knife both faces of the blade are first coated with an electrically insulating and thermally non-conducting ceramic material. Stabilized zirconia is the preferred ceramic material, although other non-conductive ceramic materials such as alumina, glasses, bioglasses and hydroxyapatite may also be used. The coating of the faces of the metallic blade is preferably carried out by plasma spray coating, either using atmospheric plasma spray coating or low pressure plasma spray coating techniques known in the art.

The plasma spraying is carried out using plasma spray torches which are generally operated using helium, neon, argon, nitrogen or hydrogen as the primary gas which is ionized to form the plasma, more preferably argon. The primary plasma forming gas may be admixed with a secondary plasma forming gas, such as a mixture of argon and hydrogen. Other thermo-spraying techniques may also be used.

Prior to coating the surfaces of the faces of the blade may be cleaned and roughened in order to provide a good surface for the deposition of the coating. The cleaning and roughening may be carried out using a shot blasting technique, for example using alumina grit under a reduced pressure.

Preferably the blade is placed flat in an appropriately designed tool, for example an aluminium or stainless steel tool which faces the plasma spray torch or torches. One face is coated with the ceramic material and the process repeated for the other face. There is no need for rotation of the blade during the coating procedure as the blade is a substantially flat object and this is an advantage for production.

The faces of the blade are coated with a thin coating of the ceramic material, preferably in the range of from 0.1 to 0.3mm, more preferably about 0.2mm. The ceramic coating generally will adhere well to the surface of the metallic blade and the coating must be sufficiently robust to withstand breakage or fracture during use of the electrosurgical knife in surgery. The coatings will typically have an open porosity in the range of from 1 to 10% by volume.

The ceramic coatings are electrically insulating under a vacuum or in a dry atmosphere. Thus, to prevent the coatings conducting electricity when in contact with physiological body fluids, such as blood, it is necessary to coat and/or impregnate the ceramic coatings with a viscoelastic polymer which is suitable for medical applications. The role of the viscoelastic material is to impregnate and seal any pores in the ceramic coating and to provide the blade with an exterior coating which does not conduct electricity when in contact with physiological fluids. Examples of viscoelastic polymers which may be used in the present invention are polytetrafluoroethylene, fluorinated ethylene-propylene copolymers, methacrylates, dimethacrylates and silicone based polymers. The viscoelastic polymer is coated on and/or impregnated into the ceramic coating using techniques which are well known in the art. The particular coating conditions will depend upon the nature of the coating which is being deposited. The surface of the blade coated and/or impregnated with the viscoelastic polymer may exhibit some roughness, but it is not necessary, in practice, for the blade to be completely smooth.

Since the faces of the blade only are coated with the ceramic material and the viscoelastic polymer the cutting edge of the blade should remain intact as no rotation of the blade is involved in the process. In practice, however, some particles of the ceramic material or of the polymeric coating may migrate onto the cutting edge of the blade. Any such particles can readily be removed by abrasion techniques which are well known in the art.

The blade of the electrosurgical knife is preferably provided with at least one connection to a source of radio-frequency electrical energy. The types of connections which may be employed will be well known to those skilled in the art. The radio-frequency source to which the blade will be attached during use will generally be of variable power in order that the electrosurgical knife may be controlled by the surgeon during use.

The electrosurgical knife of the present invention possesses the following advantages:
i) the current density is concentrated onto the cutting edge of the blade;
ii) the heat produced during use is dissipated in the steel, but because of the thermal barrier it does not reach the surface of the blade;
iii) because the flat coated surfaces of the blade remain cold, the blade does not stick to and char tissues; and
iv) the cutting edge is easy to clean during surgery using an abrasive instrument.

These advantages lead to an appropriate marked reduction of up to 50% in the power necessary to use the electrosurgical knife. Accordingly, a knife which would typically operate at 35 to 50 watts could be operated at about 20 to 25 watts.

The present invention will be further described with reference to the following Example.

### EXAMPLE

An electrosurgical knife was sand blasted on those surfaces which are not used for cutting or cauterizing tissues. The sand blasting was carried out with F60 Al₂O₃ grains from a distance of 5 to 15cm under a pressure of 4 bar. The knife was then cleaned for 20 minutes in an ultrasonic bath with deionised water to remove all of the debris resulting from the sand blasting operation. After cleaning the knife was dried in a hot air stream and inspected for defects. An alumina layer was then deposited by a plasma coating technique under the following conditions:-
- atmospheric plasma
- power 40KW (470A, 85V)
- H₂ : 30 litres/min
- Ar : 40 litres/min
- flow of powder feed: N₂ 7 litres/min
- distance between gun and target (knife) = 9cm

The ceramic material used was an alumina powder with a grain size of 22-45cm. The layer thickness was 0.2mm.

After cooling, the knife was dipped in a Teflon top coat type 852-200 (Dupont) for approximately 10 seconds and centrifuged at 9,000 rpm to remove the excess of polymer. The knife was then dried in a furnace at 70°C for 10 hours. When this operation was complete the Teflon coating was cured at 385°C for 30 minutes. The debris of ceramic material was removed from the cutting edge of the knife using sand paper (grit P320).

## Claims

1. An electrosurgical instrument for use with a source of radio-frequency energy during surgery to cut or to cauterize tissue, which instrument comprises a metallic tool portion which is coated on the faces thereof, other than the edge or area which is in use during surgery, with an electrically insulating and thermally non-conductive ceramic material which is coated and/or impregnated with a viscoelastic polymer.

2. An electrosurgical instrument as claimed in claim 1 which is an electrosurgical knife.

3. An electrochemical instrument as claimed in claim 1 or claim 2 wherein the ceramic material is stabilized zirconia or alumina.

4. An electrosurgical instrument as claimed in any one of the preceding claims wherein the ceramic material is from 0.1 to 0.3mm thick.

5. An electrosurgical instrument as claimed in any one of the preceding claims wherein the viscoelastic polymer is polytetrafluoroethylene or a fluorinated ethylene-propylene copolymer.

6. An electrosurgical instrument as claimed in any one of the preceding claims wherein the metallic tool portion is provided with at least one connection to a source of radio-frequency electrical energy.

7. A method for the preparation of an electro-surgical instrument for use with a source of radiofrequency energy during surgery to cut or cauterize tissue, which method comprises coating metallic tool portion of the said instrument on the faces thereof, other than the edge or area which is in use during surgery, with an electrically insulating and thermally non-conductive ceramic material, and coating or impregnating the said coating of the ceramic material with a viscoelastic polymer.

8. A method as claimed in claim 7 wherein the ceramic material is alumina.

9. A method as claimed in claim 7 or claim 8 wherein the alumina is deposited upon the faces of the metallic tool portion by plasma spraying.

10. A method as claimed in any one of claims 7 to 9 wherein the ceramic material has a thickness of from 0.1 to 0.3mm.

11. A method as claimed in any one of claims 7 to 10 wherein the viscoelastic polymer is polytetrafluoroethylene or a fluorinated ethylene-propylene copolymer.

12. A method as claimed in any one of claims 7 to 11 wherein any ceramic material or viscoelastic material coating the cutting edge of the blade of an electrosurgical knife is removed therefrom.
